Europäisches Patentamt

European Patent Office

Office européen des brevets

(19)

(11) Veröffentlichungsnummer: **0 263 096**
**B1**

(12) **EUROPÄISCHE PATENTSCHRIFT**

(45) Veröffentlichungstag der Patentschrift:
29.03.89

(21) Anmeldenummer: 86900768.2

(22) Anmeldetag: 21.01.86

(86) Internationale Anmeldenummer:
PCT/EP 86/00020

(87) Internationale Veröffentlichungsnummer:
WO 86/06951 (04.12.86 Gazette 86/26)

(51) Int. Cl.⁴: **A 61 B 10/00**

(54) Vorrichtung und Verfahren zur Entnahme von Flüssigkeiten, Gewebe.

(30) Priorität: 23.05.85 DE 3518547

(43) Veröffentlichungstag der Anmeldung:
13.04.88 Patentblatt 88/15

(45) Bekanntmachung des Hinweises auf die Patenterteilung:
29.03.89 Patentblatt 89/13

(84) Benannte Vertragsstaaten:
AT BE CH DE FR GB IT LI LU NL SE

(56) Entgegenhaltungen:
GB-A-2 000 976
US-A-3 035 616
US-A-4 177 814
US-A-4 230 123

(73) Patentinhaber: Schnepp- Pesch, Wolfram,
Schönblick 6, D-7505 Ettlingen (DE)
Patentinhaber: Lindenberg, Josef, Buchenweg 13,
D-7512 Rheinstetten 4 (DE)

(72) Erfinder: Schnepp- Pesch, Wolfram, Schönblick 6,
D-7505 Ettlingen (DE)
Erfinder: Lindenberg, Josef, Buchenweg 13, D-7512
Rheinstetten 4 (DE)

(74) Vertreter: Dr.- Ing. Hans Lichti Dipl.- Ing. Heiner
Lichti Dipl.- Phys. Dr. Jost Lempert, Postfach 41
07 60 Durlacher Strasse 31, D-7500 Karlsruhe 41
(DE)

EP 0 263 096 B1

LIBER, STOCKHOLM 1989

**Beschreibung**

Die Erfindung betrifft eine Vorrichtung mit einem gestreckten Hohlkörper, wobei in einem einem Einführende abgewandten Bereich des Hohlkörpers eine Dichtung angeordnet ist und durch diese hindurch und im Hohlkörper ein an dessen Hohlraum im wesentlichen angepaßter, gestreckter Obturator geführt ist, ein Verfahren zur Entnahme von Flüssigkeiten, Gewebe oder dergleichen sowie die Verwendung der genannten Vorrichtung in einem entsprechenden Verfahren.

Es sind beispielsweise Biopsiebestecke mit einer Hohlnadel als Hohlkörper bekannt, die an einem in den Körper einzuführenden Ende, in geeigneter Form zugeschliffen ist, wobei weiterhin zumindest während des Einstechens sich in der Hohlnadel ein Mandrin befindet, der an seinem vorderen distalen Ende stilettartig zugespitzt sein kann und auch als Stilett bezeichnet wird. An seinem, rückwärtigen, proximalen Ende, kann er einen Handgriff aufweisen. Wenn das distale Ende in den Bereich gelangt ist, in dem die Gewebeprobe entnommen wird, wird der Mandrin zumindestens teilweise oder auch ganz herausgezogen und eine Gewebeprobe zur histologischen und/oder zytologischen Untersuchung durch weiteres Einstechen oder druckloses Schneiden entnommen, wobei zum zusätzlichen Aufsaugen des Bioptats nach Einstechen der Stahlnadel und anschließendem Entfernen des Mandrins, eine Medizinalspritze befestigt wird. Anschließend wird die Spitze aufgezogen, die gesamte Anordnung gedreht, um einen Materialzylinder herauszuschneiden und schließlich die Anordnung aus dem Körper herausgezogen und der Materialzylinder aus der Hohlnadel mittels Herunterdrücken der Spritze ausgestoßen. Das zur Entnahme beschriebene Vorgehen kann leicht zu einer Verschiebung der Hohlnadel führen, die zur Fehlleitung mit Komplikationen und zur Schmerzverursachung führen kann. Bis zum Aufsetzen der Medizinalspritze steht das beispielsweise bei einer Lungenpunktion in die Lunge eingeführte Ende in nachteiliger Weise mit dem Außenluftraum in Verbindung. Die Gewebeflüssigkeit kann mit diesem Biopsiebesteck nicht entnommen werden. Insbesondere hierzu ist ein anderes Biopsiebesteck vorgesehen, bei dem die Hohlnadel am Zylinder einer Medizinalspritze und ein in der Hohlnadel geführtes Hohlstilett am Kolben der Spritze befestigt ist, so daß beim Aufziehen der Spritze Gewebeflüssigkeit durch den entstehenden Unterdruck in den Zylinder der Spritze eingesaugt wird. Diese Spritze weist den Nachteil auf, daß aufgrund des beim Aufsaugen im Zylinder erzeugten großen Volumens eine relativ große Gewebeflüssigkeitsmenge aufgesaugt wird, die zur Traumatisierung des untersuchten Gewebes führen kann. Dennoch ist aufgrund des relativ großen Ausgangsvolumens vor dem Aufsaugen schon beim Beginn des Aufsaugens ein relativ schwacher Unterdruck vorhanden, der nachteilig sein kann.

Die US-A-4 230 123 zeigt ein gattungsgemäßes Biopsiebesteck mit Biopsiehohlnadel. Ein Gummi-Ring umgibt die Hohlnadel und dichtet sie gegen eine weitere sie umgebende Hülse ab. Der Innenraum der Hohlnadel ist gegen die Atmospähre offen.

Die GB-A-2 000 976 betrifft lediglich eine Verbindung für eine Spritze zu einem Katheter, wobei der Katheter oder die Nadel lösbar in einem Adapter anbringbar ist. Eine Dichtung umgibt die Hohlnadel. Das Innere der Hohlnadel steht, wenn mit der Umgebung oder mit dem Innenraum einer aufgesetzten Spritze in Verdung.

Die US-A-4 177 814 betrifft eine nicht gattungsgemäße Vorrichtung zum Aufblasen von Körperhohlräumen mit unter Druck stehendem Gas, mit einer Hülse durch die Geräte wie Endoskope, Laproskope oder dergleichen einführbar sein sollen. Die Hülse weist eine topfförmige Dichtkappe mit sternförmigen Schlitzen oder Linschnitten auf, um das einzuführende Teil einbringen zu können. Derartige Vorrichtungen sind aus der Praxis mit zentimeterstarken Hülsen bekannt. Zum Aufblasen genügt ein im Verhältnis zum Atmosphärendruck geringer Überdruck, wobei eine Pumpeinrichtung in der Praxis auftretende Druckverluste permanent aufrecht erhält. Das Festhalten einer Hohlnadel in einem Adapter ist auch Gegenstand der US-PS-3 035 616.

Der Erfindung liegt die Aufgabe zugrunde, eine Vorrichtung der gattungsgemäßen Art derart weiter zu bilden, daß mittels diesen in definierter und schonender Weise Gewebe oder Flüssigkeit in einfacher, insbesondere in einfach handhabbarer Weise derart entnommen werden kann, daß die Entnahme effizient und kürzer erfolgt und gegebenenfalls für einen Patienten weniger anstrengend ist sowie durch geringere Manipulationen die Traumatisierungsgefahr reduziert wird und Treffsicherheit an Entnahmestellen.

Erfindungsgemäß wird die genannte Aufgabe einer gattungsgemäßen Vorrichtung durch die Merkmale des Anspruchs 1 gelöst. Zum Einsatz bei totem Material, wie totem Gewebe sieht die Erfindung ein Verfahren nach dem Anspruch 10 und eine Anwendung nach dem Anspruch 13 vor.

Sowohl Gewebeproben zurhistologischen Untersuchung als auch Gewebeflüssigkeit zur zytologischen Untersuchung oder auch sonstige Flüssigkeiten werden bei der erfindungsgemäßen Vorrichtung in dem beim Aufziehen des Obturators im Hohlkörper freigegebenen Hohlraum mit starker Saugkraft eingesogen und aufgenommen. Während die erfindungsgemäße Vorrichtung insbesondere zur Biopsie an Lebewesen vorgesehen ist, kann sie auch zur postmortalen Biopsie und zur Entnahme von Proben aus sonstigen geeigneten toten Material, wie zur Werkstoffprüfung eingesetzt werden. Das verfahrensmäßige Vorgehen gestaltet sich mit der erfindungsgemäßen Vorrichtung wesentlich einfacher. Nach Einführen des länglichen Hohlkörpers, mit eingeführtem Obturator in den Körper, wird der Obturator aufgezogen und gegebenenfalls vollständig entfernt, wobei im letzteren Falle die Dichtung selbstschließend ausgebildet ist, so daß sie sich nach Entfernen des Obturators vollständig schließt und daher Unterdruck im Hohlkörper aufrechterhalten bleibt. Damit wird gegebenenfalls aufgesogene Flüssigkeit im Höhlkörper gehalten und kann mit diesem aus dem Körper entfernt werden. Bei Punktieren zur Gewebeentnahme mittels einer Hohlnadel mit eingeführten Mandrin, wird letzteres aufgesogen und gegebenenfalls vollständig entfernt, wodurch ebenfalls der erzeugte Unterdruck aufrechterhalten wird. Die Hohlnadel braucht lediglich noch über die Länge des gewünschten Gewebepfropfens eingestoßen werden, während ein Eindrehen zum Herausschneiden nicht mehr unabdingbar ist, das Herausschneiden vielmehr beim leichten Einstoßen durch den Unterdruck bewirkt wird. Nach Herausziehen der Hohlnadel aus dem Körper wird die Gewebeprobe durch erneutes Einführen und/oder Einstoßen des Mandrins aus der Hohlnadel ausgestoßen, wobei die Gewebeproben bei jeglicher Konsistenz des Gewebes, sei es z. B. Lungen-, Nieren- oder Lebergewebe im Gefüge wesentlich besser erhalten bleiben. Dies ergibt sich daraus, daß bei der Entnahme einer Gewebeprobe beim Ausschneiden oder Ausstechen des Gewebepfropfens aus dem zu untersuchenden Gewebebereich die hierbei erfolgende Aufnahme des Gewebes durch den Unterdruck in der Hohlnadel unterstützt, der aus dem Gewebe herausgeschnittene Gewebepfropfen in die Hohlnadel eingesaugt wird, so daß es nicht zu einer Stauchung desselben kommt. Es entfällt eine nachteilige Traumatisierung des Bioptats, da der ausgeschnittene Gewebszylinder nicht in eine Spritze hineinschießen kann. Dadurch, daß die Abdichtung zwar mit Abstand zum in den Körper eingeführtes Ende der Hohlnadel und vorzugsweise im Bereich des abgewandten Endes, aber im Inneren derselben, gegebenenfalls im Inneren eines erweiterten Ansatzes oder dergleichen ausgebildet ist, ergibt sich der Vorteil, daß nach Zurückziehen des Mandrin auch bei der Entnahme von Gewebeflüssigkeit eine wohldefinierte Menge derselben mit höherem Unterdruck als beim Stand der Technik in den freigegebenen Bereich der Hohlnadel eingesaugt wird, dann aber von einem durch die vor Aufziehen des Obturators gegebene Restkraft gegebenen Luftpolster vor der Dichtung gefedert abgefangen wird. Diese wohlverdefinierte geringe, aber zur Untersuchung ausreichende Menge führt nicht zu einer übermäßigen Traumatisierung des entnommenen Gewebes. Die erfindungsgemäße Hohlnadel führt also auch daher bei ihrer Benutzung zu geringerer Belastung für den Patienten. Grundsätzlich wird durch die erfindungsgemäße Ausgestaltung die Durchführung und Ausbeute - nämlich eines längeren intakten Gewebezylinders - der Biopsie aufgrund ihrer einfachen Handhabung verbessert. Es hat sich überraschender Weise herausgestellt, daß die erfindungsgemäße Vorrichtung eine wesentlich größere Saugkraft am distalen Ende des Hohlkörpers entfaltet, als dies bei Einsatz üblicher Medizinalspritzen zur Erzeugung des Vakuums der Fall ist. Dies dürfte daran liegen, daß aufgrund des üblicherweise dicht einsitzenden Obturators im Hohlkörper - beide sind mit geringen Toleranzen aufeinander abstimmbar -, das Verhältnis von Restluft enthaltendem Restvolumen vor dem Aufziehen des Obturators zum beim Aufziehen sich ergebenden Saugvolumen sehr gering bzw. der resiprohe Wert groß ist.

Durch die Dichtung kann kein Material gesaugt werden, denn auch nach Entfernen des Mandrins ist der Hohlkanal wieder verschlossen. Eine Medizinalspritze ist im Gegensatz zum Stand der Technik nicht mehr unabdingbar. Es ergibt sich eine bessere Zielgenauigkeit und größere Feinfühligkeit, z. B. bei einem Einstoßen aber auch bei einem Drehen der Hohlnadel zum Drehschneiden. Insbesondere können alle verschiedenartigen Schneidausbildungen am distalen Ende der Hohlnadel mit der erfindungsgemäßen Ausgestaltung kombiniert werden.

Gemäß bevorzugter Ausgestaltung ist vorgesehen, daß die Dichtung aus Gummi besteht oder eine Silikondichtung oder anderes Material ist. Die Dichtung wird als vollständig geschlossene Dichtscheibe in das Innere des Hohlkörpers eingesetzt und dann durch den Mandrin durchstochen, wobei die Dichtung diesen vollständig und zuverlässig dicht umgibt. Die Dichtung bleibt bei teilweisem Herausziehen bzw. Entfernen des Obturators zwangsläufig dicht. Soll der Obturator oder Mandrin vollständig entfernt werden, so bei vollständigem Herausziehen des Mandrins, bleibt die Dichtscheibe durch Selbstdichtung dicht, dichtet also den Hohlraum der Nadel wieder vollständig ab und hält derart das die Saugwirkung schaffende Vakuum im Hohlkörper aufrecht. In bevorzugter Weiterbildung ist vorgesehen, daß der Hohlkörper einen erweiternden Hohlraum, beispielsweise im Inneren eines Adapters wie eines Luer-Adapters aufweist und daß die Dichtung im erweiterten Hohlraum ausgebildet ist. Bei einer derartigen Ausgestaltung ist die Dichtung verfahrensmäßig einfacher einzubringen, wobei darüberhinaus der weitere Vorteil gegeben ist, daß durch den größeren Durchmesser der Dichtung gegenüber dem durch sie hindurch gestochenen Mandrin mehr Dichtungsmasse zur Verfügung steht, um beim vollständigen Herausziehen des Mandrins wieder zu einer zuverlässigen Abdichtung zu gelangen. Die Dichtung wird dabei in vorteilhafter Weise durch einen Haltering oder auch durch Einkleben festgehalten. Neben den vorgenannten Vorteilen beim Einsatz und Gebrauch der erfindungsgemäßen Hohlnadel, ergibt der Umstand, daß keine Medizinalspritze zum Aufziehen des Mandrins oder Stiletts eingesetzt werden muß - und zur optimalen Nutzung aller Vorteile der Erfindung auch nicht verwendet werden sollte -, noch den Vorteil einer Materialersparnis und damit größerer Preiswürdigkeit. Die erfindungsgemäße Vorrichtung kann insbesondere auch zum Einsatz mit einem Endoskop vorgesehen und erforderlichenfalls entsprechend ausgebildet sein. Aufgrund des starren Krümmens herkömmlicher Endoskope kann hierzu die Vorrichtung einen angular hinreichend flexiblen axial aber möglichst steifen Führungsschlauch zum Einführen durch das Endoskop aufweisen. Der Schlauch ist vorzugsweise als Metallschlauch mit einem starren vorderen, also distalen, geschärften, also als Hohlnadel ausgebildeten Ende, dessen Länge in Abhängigkeit seines Durchmessers von den Abmessungen der Endoskopkrümmung bestimmt ist. Der Metallschlauch kann beispielsweise durch eine Schraubfeder oder in anderer geeigneter Weise ausgebildet sein. Erforderlichenfalls weist er zur Sicherstellung des erforderlichen Unterdruckes im Entnahmebereich eine an der Übergangsstelle zwischen flexiblem Schlauch und starren Hohlnadel im Inneren eine Ringdichtung auf. Zur Kontrolle der Aufziehlänge kann am proximalen Betätigungsende eine Markierung oder ein Anschlag vorhanden sein.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnung im einzelnen erläutert ist. Dabei zeigt:

Fig. 1     eine erste Ausführungsform eines Biopsiebestecks mit einer bevorzugten Ausgestaltung der erfindungsgemäßen Hohlnadel;

Fig. 2     eine Übersichtsdarstellung eines anderen Biopsiebestecks mit der bevorzugten Ausführungsform der erfindungsgemäßen Hohlnadel;

Fig. 3     eine Teildarstellung der Figur 2 mit herausgezogenem Kolben; und

Fig. 4     eine Detaildarstellung des Bereichs III der Figur 2, vollständig geschnitten;

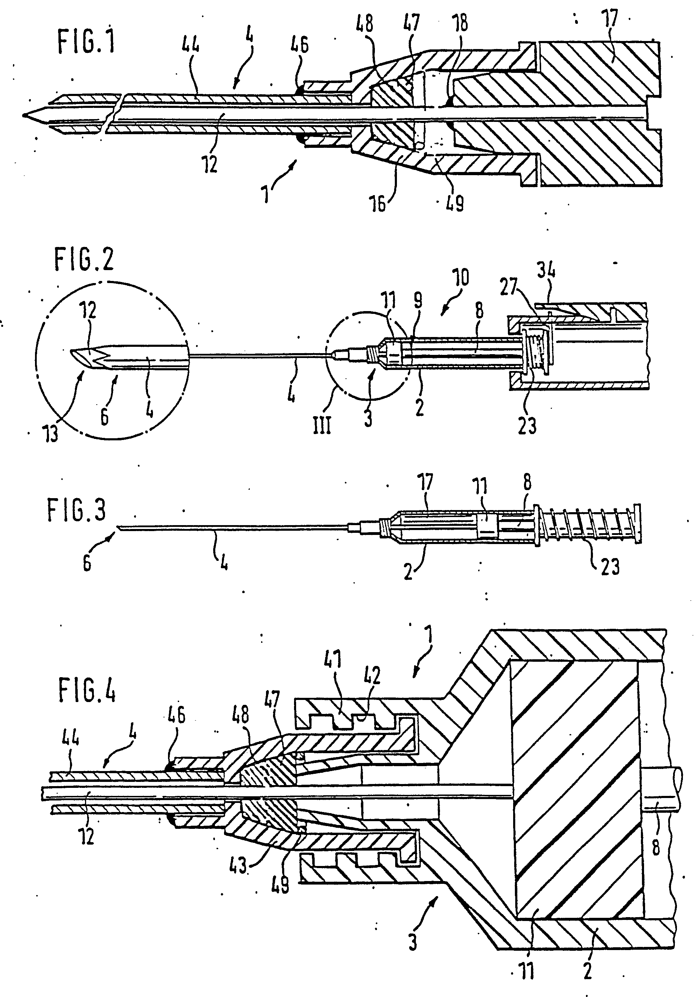

Das in der Figur 1 dargestellte Biopsiebesteck 1 weist eine Kanüle oder Hohlnadel 4 auf, die aus einem Metallschaft 44 und einem Griffteil 16 besteht, in dem der Metallschaft 44 fest eingesetzt und beispielsweise mittels einer Vergußmasse 46 verbunden ist. Das Griffteil 16 besteht aus einem geeigneten stabilen Kunststoff und kann beispielsweise in Form eines Adapters, wie eines Luer-Adapters ausgebildet sein, hat aber auch dann in der in Figur 1 dargestellten Ausführungsform die Funktion eines Griffteils, könnte dann aber auch bei der in den Figuren 2 bis 4 dargestellten Ausführungsform eingesetzt werden. Das Griffteil 16 könnte selbstverständlich auch anders ausgestaltet sein, als dies in Figur 1 der Fall ist, beispielsweise mit radial abstehenden Flanschen etc. Weiterhin weist das Biopsiebesteck 1 ein Stilett oder Mandrin 12 auf, welches in der Hohlnadel 4 geführt ist. Hohlnadel 4 und Mandrin 12 sind an ihren dem Griffteil 16 abgewandten Ende angespitzt und geschärft, beispielsweise die Hohlnadel 4 mit Trokarschliff, Innenschliff oder dergleichen. Der Mandrin 12 weist an seinem proximalen Ende eine Handhabe 17 auf, die ebenso wie das Griffteil 16 aus stabilen Kunststoff, vorzugsweise dem gleichen Kunststoff besteht und in der der Mandrin fest eingesetzt und beispielsweise mittels Vergußmasse 18 festgelegt ist. Bei voll in die Hohlnadel 4 eingefahrenem Mandrin 12, ragt die Spitze des Mandrins 12 aus dem in den Körper einzuführenden Ende der Hohlnadel heraus. Im Griffteil 16 der Hohlnadel 4, ist eine Dichtung 47 eingesetzt. Sie ruht einerseits auf eine im Inneren des Griffteils 16 ausgebildeten Schulter 48 und wird andererseits auf ihre der Schulter 48 gegenüberliegenden Seite durch einen eingesetzten und mit dem Griffteil 16 fest verbundenen, beispielsweise verschweißten Ring 49 gehalten. Die Dichtung 47 ist vorzugsweise eine Scheibe aus geeignetem, elastischen Material, wie Kunststoff, Gummi, Kautschuk, z. B. Silikonkautschuk oder dergleichen. Beim Einführen des Mandrins 12 in die Hohlnadel 4 wird die Dichtungsscheibe 47 durchstoßen und umgibt dann den Mandrin 12 mit einer außerordentlich hohen Dichtigkeit. Auch das Biopsiebesteck wird zur Entnahme von insbesondere histologisch zu untersuchenden Gewebe aus Körperorganen eingesetzt, in die die Hohlnadel 4 mit eingeführten Mandrin 12 (also in der Darstellung der Figur 1) ins Körperinnere - gegebenenfalls unter Zuhilfenahme einer Führungsnadel, wie einer Strauskanüle - eingeführt wird. Zur Entnahme wird unter Festhalten der Hohlnadel 4 an dessen Griffteil 16 der Mandrin 12 nach Angreifen an dessen Handhabe 17 aufgezogen und damit der vordere Hohlraum der Hohlnadel 4 zur Aufnahme von Gewebeteilen freigegeben, die schon etwas ins distale Ende der Hohlnadel 4 eingesogen werden. Insbesondere kann der Mandrin 12 auch vollständig entfernt werden, wobei die Dichtung 47 dann den Innenraum der Hohlnadel 4 selbsttätig zuverlässig abdichtet. Bei der Aufnahme von Gewebe wird insbesondere bei einem geeigneten Schliff des eingeführten Endes der Hohlnadel 4, diese durch Drehen oder alleiniges Drücken am Griffteil 16 in Bewegung versetzt und weiter in dem zu interessierendem Bereich eingedrückt, so daß insgesamt eine schraubende Bewegung der Hohlnadel 4 entsteht, wodurch ein Gewebepfropfen als Bioptat aus dem Gewebe herausgeschnitten und in die Hohlnadel 4 voll eingesogen wird. Die Hohlnadel 4 wird mit dem Bioptat aus dem Körper herausgesogen und anschließend das Bioptat mittels des erneut in die Hohlnadel 4 eingeschobenen Mandrins 12 aus der Hohlnadel aus und zur Fixierung z. B. in Formalin eingeschoben.

In grundsätzlich entsprechender Weise, kann mit dem beschriebenen und in der Figur 1 dargestellten Biopsiebestech mit der erfindungsgemäßen Hohlnadel 4 auch eine wohl definierte Menge Gewebeflüssigkeit zur zytologischen Untersuchung aufgesogen werden, ohne daß hierzu eine Medizinalspritze erforderlich wäre, wenn eine solche aber auch, wie weiter unten dargestellt, falls dies aus anderen Gründen gewünscht wird, eingesetzt werden kann. In diesem Falle unterbleibt das Herausschneiden von Gewebeteilen durch die Hohlnadel und es wird bei festgehaltener Hohlnadel 4 lediglich durch Aufziehen des Mandrins 12 über die Handhabe 17 Gewebeflüssigkeit in die Hohlnadel 4 eingesogen, wobei die Menge mit hoher Genauigkeit durch den Betrag des Aufziehens bestimmt werden kann. Im folgenden wird eine weitere Ausgestaltung eines Biopsiebestecks mit grundsätzlich der gleichen Ausführungsform der erfindungsgemäßen Hohlnadel beschrieben, wobei bei prinzipiell gleichen Teilen gleiche Bezugszeichen verwendet werden.

Das in den Figuren 2 bis 4 dargestellte Biopsiebesteck 10 weist eine Medizinalspritze mit einem Hohlzylinder 2 auf, mit dem an einem vorderen Ende 3 die Kanüle oder Hohlnadel 4 verbunden ist. Das Ende 3 des Hohlzylinders 2, kann dabei einen Verbindungsansatz 41 mit einem Innengewinde 42 aufweisen, an dem die Hohlnadel 4 mittels eines Adapters 43 beispielsweise in der Art eines Luer-Adapters befestigbar ist,der grundsätzlich wie das Griffteil 16 der Figur 1 ausgebildet sein kann, aber hier nicht als solche dient. In dem Hohlzylinder 2 ist ein Kolben 8 geführt, der an seinem der Hohlnadel 4 zugewandten Ende einem im Hohlzylinder sitzenden Stempel 11 aufweist. Mit dem Kolben 8 ist ein in der Hohlnadel 4 geführtes Stilett oder Mandrin 12 verbunden. Hohlnadel 4 und Mandrin 12 sind an ihrem Hohlzylinder 2 bzw. Kolben 8 abgewandten Enden 6, 13 angespitzt und geschärft, beispielsweise die Hohlnadel 4 mit Trokarschliff, Innenschliff oder dergleichen. Bei voll in den Hohlzylinder 2 eingefahrenem Kolben 8, ragt die Spitze des Stiletts 12 wenige Millimeter aus dem Ende 6 der Hohlnadel 4 heraus. Der Kolben 8 kann entweder von Hand im Hohlzylinder 2 aufgezogen werden oder aber unter der Spannung einer Feder 23 stehen und zunächst mittels einer mit dem Hohlzylinder 2 verbundenen Arretiereinrichtung 27 stehen, die über ein Betätigungsglied 34 gelöst werden kann, wie dies in der Figur 1 dargestellt ist.

Die Hohlnadel 4 weist, wie gesagt, ein Adapterteil 43 auf, das mit dem Metallschaft 44 der Hohlnadel 4 fest verbunden ist, beispielsweise mittels einer Vergußmasse 46. Im Adapterteil 43 der Hohlnadel 4, ist - wie oben bei Figur 1 beschrieben - eine Dichtung 47 eingesetzt. Die Dichtung ist wie die der Ausführung nach Figur 1 ausgebildet und festgelegt.

Dieses Biopsiebesteck wird ebenfalls zur Entnahme von insbesondere histologisch zu untersuchenden Gewebe aus Körperorganen, zytologisch zu untersuchen oder Gewebeflüssigkeit eingesetzt. Der Einsatz erfolgt dabei grundsätzlich in der zur Figur 1 beschriebenen Weise, wobei entsprechend am Zylinder 2 und Kolben 8 angegriffen wird bzw. der Kolben durch Freigabe einer unter Spannung stehenden Feder aufgezogen werden kann.

Die erfindungsgemäße Vorrichtung kann auch einen Kunststoffhohlkörper aufweisen, in dem ein Obturator mit einer Abdichtung, wie sie unter Bezug auf die Figur 1 beschrieben wurde, geführt ist. Das der Einführöffnung des Obturators entgegengesetzte in den Körper einzuführende Ende des Kunststoffhohlkörpers kann geschlossen sein, während in diesem Bereich in der Seitenwand Saugöffnungen angeordnet sind. Statt dessen könnte je nach Einsatzzweck auch die Stirnseite selbst offen sein.

## Patentansprüche

1. Vorrichtung mit einem gestreckten Hohlkörper, wobei in einem einem Einführende abgewandten Bereich des Hohlkörpers eine Dichtung angeordnet ist und durch diese hindurch und im Hohlkörper (1) ein an dessen Hohlraum im wesentlichen angepaßter, gestreckter Obturator geführt ist, dadurch gekennzeichnet, daß zur Entnahme von Flüssigkeiten, Gewebe oder dergleichen aus dem Inneren eines Körpers, wie eines Lebewesens, eine Saugeinrichtung aus Hohlkörper und Obturator dadurch gebildet ist, daß der Hohlkörper eine Hohlnadel (4) mit am distalen Ende (6) ausgebildeter Schneide und der Obturator ein in der Hohlnadel geführter Mandrin (12) ist und daß die Dichtung als vollständig geschlossene Dichtscheibe (47) eingesetzt ist, die ausschließlich erst durch den Mandrin (12) durchstochen ist.

2. Vorrichtung nach Anspruch 1, dadurch gekennzeichnet, daß die Dichtscheibe (47) aus Gummi besteht.

3. Vorrichtung nach Anspruch 1, dadurch gekennzeichnet, daß die Dichtscheibe (47) aus Kunststoff besteht.

4. Vorrichtung nach Anspruch 3, dadurch gekennzeichnet, daß die Dichtscheibe (47) aus Silikon besteht.

5. Vorrichtung nach einem der vorangehenden Ansprüche, dadurch gekennzeichnet, daß der Hohlkörper im Bereich seines in den Körper einzuführenden Endes abgewandten Ende einen erweiterten Hohlraum, beispielsweise im Inneren eines Adapters (43), wie eines Luer-Adapters, aufweist und daß die Dichtung (47) im erweiterten Hohlraum ausgebildet ist.

6. Vorrichtung nach Anspruch 5, dadurch gekennzeichnet, daß die Dichtung (47) ihren Aufnahmehohlraum hohlnadelseitig vollständig ausfüllt.

7. Vorrichtung nach einem der vorangehenden Ansprüche, dadurch gekennzeichnet, daß die Dichtung (47) durch einen Haltering (49) gehalten wird.

8. Vorrichtung nach einem der vorangehenden Ansprüche, dadurch gekennzeichnet, daß die Dichtung (47) durch eine insbesondere ringförmige Ultraschallschweißstelle gehalten ist.

9. Vorrichtung nach einem der vorangehenden Ansprüche, dadurch gekennzeichnet, daß die Dichtung nach Durchstechen selbstschließend ist.

10. Verfahren zur Entnahme von Flüssigkeiten, Gewebe oder dergleichen aus dem Inneren toten Materials, dadurch gekennzeichnet, daß in den Körper bis zum Entnahmegebiet das distale Ende einer Saugeinrichtung eingestochen wird, die eine Hohlnadel mit an ihrem distalen Ende ausgebildeter Schneide und einen den Hohlraum der Hohlnadel im wesentlichen angepaßten Mandrin und in einem distalen Ende der Hohlnadel abgewandten Bereich, eine als vollständig geschlossene Dichtscheibe eingesetzte Dichtung aufweist, die ausschließlich erst durch den Mandrin durchstochen ist und daß anschließend der Mandrin im Hohlkörper zumindestens teilweise zurückgezogen und dabei Gewebeflüssigkeit oder Gewebeproben in die Hohlnadel eingesogen werden.

11. Verfahren nach Anspruch 10, dadurch gekennzeichnet, daß beim Zurückziehen des Mandrins die Hohlnadel gleichzeitig etwas vorgestoßen wird.

12. Verfahren nach Anspruch 10 oder 11, dadurch gekennzeichnet, daß nach Herausziehen der Hohlnadel aus dem Körper eine entnommene Biopsieprobe durch Einstoßen des Mandrins, gegebenenfalls nach erneutem Einführen durch die Dichtung der Hohlnadel ausgestoßen wird.

13. Verwendung einer Vorrichtung, die als Saugeinrichtung dadurch ausgebildet ist, daß in einer Hohlnadel (4) mit am distalen Ende (6) ausgebildeter Schneide ein an den Hohlraum derselben im wesentlichen angepaßter, gestrechter Mandrin (12) geführt ist, daß in einem dem distalen Ende (6) der Hohlnadel (4) abgewandten Bereich eine Dichtung in Form einer vollständig geschlossenen Dichtscheibe (47) eingesetzt ist, die ausschließlich erst durch den Mandrin (12) durchstochen ist, zur Entnahme von Flüssigkeiten, Gewebe oder dergleichen aus dem Inneren toten Materials.

**Claims**

1. Apparatus with an elongated hollow body, a seal being arranged in an area of the hollow body remote from an insertion end and through said seal and in hollow body (1) is guided an elongated obturator substantially adapted to the cavity of the hollow body, characterized in that for use in removing liquids, tissue or the like from the interior of a body, such as a living organism, a suction device is made by the hollow body and the obturator in that the hollow body is a hollow needle (4) with cutting edge formed at the distal end (6) and the obturator is a mandrel (12) guided in the hollow needle and that the seal is inserted as a completely closed sealing disk (47), which is exclusively perforated by the mandrel (12).

2. Apparatus according to claim 1, characterized in that the seal (47) is made from rubber.

3. Apparatus according to claim 1, characterized in that the seal (47) is made from plastic.

4. Apparatus according to claim 3, characterized in that the seal (47) is made from silicone.

5. Apparatus according to one of the preceding claims, characterized in that in the vicinity of its end remote from that to be inserted in the body, the hollow body has a widened cavity, for example in the interior of an adapter (43), such as a Luer adapter and that the seal (47) is formed in the widened cavity.

6. Apparatus according to claim 5, characterized in that on the cannula side, seal (47) completely fills its reception cavity.

7. Apparatus according to one of the preceding claims, characterized in that the seal (47) is secured by a retaining ring (49)

8. Apparatus according to one of the preceding claims, characterized in that the seal (47) is secured by an in particular annular ultrasonic welding point.

9. Apparatus according to one of the preceding claims, characterized in that the seal is self-sealing.

10. Method for removing liquids, tissue or the like from the interior of dead material, characterized in that the distal end of a suction device is inserted into the body up to the removal region the suction device having a hollow needle with a cutting edge formed at its distal end and a mandrel substantially adapted to the cavity of the hollow needle and in an area remote from the distal end of the hollow needle a seal inserted as a completely closed sealing disk, which is exclusively perforated by the mandrel and that subsequently the mandrel is at least partly retracted in the hollow body and thereby tissue fluid or samples are sucked into the hollow needle.

11. Method according to claim 10, characterized in that in withdrawing the mandrin simultaneausly the cannula is advanced.

12. Method according to claimes 10 or 11, characterized in that following the step of removing the cannula from the body the biopsy sample taken is expelled by advancing the mandrin in eventually reinserting it through the sealing.

13. Use of an apparatus constructed as a suction device in that in a hollow needle (4) with a cutting edge formed at the distal end (6) is guided an elongated mandrel (12) substantially adapted to the cavity thereof and in that an area remote from the distal end (6) of the hollow needle (4) is inserted a seal in the form of a completely closed sealing disk (47), which is exclusively perforated by the mandrel (12) for removing liquids, tissue or the like from the interior of the dead material.

**Revendications**

1. Dispositif muni d'un corps creux ablong, où un joint d'étanchéité est aménagé dans une zone du corps creux, écarté d'extrémité d'introduction, au travers du joint et dans le corps creux (1) un obturateur oblong etant introduit, qui est adapté de manière essentielle au creux du corps, caractérisé en ce que pour le prélèvement de liquides, de tissus ou similaires de l'intérieur d'un corps, comme par exemple un être vivant, un dispositif aspirateur est formé par le corps creux et l'obturateur de telle manière que le corps creux est une aiguille creuse (4) portant un tranchant à son extrémité distale (6) et l'obturateur est un mandrin (12) guidé dans l'aiguille creuse et que le joint d'étanchéité est une rondelle d'étanchéité (47) qui n'est piquée que par le mandrin (12).

2. Dispositif selon la revendication 1, caractérisé en ce que la rondelle d'étanchéité (47) est en caoutchouc.

3. Dispositif selon la revendication 1, caractérisé en ce que la rondelle d'étanchéité (47) est en matière synthétique.

4. Dispositif selon la revendication 1, caractérisé en ce que la rondelle d'étanchéité (47) est en silicone.

5. Dispositif selon une des revendications précédentes, caractérisé en ce que le corps creux présente un creux élargi à l'endroit de son extrémité, opposée à l'extrémité à introduire dans le corps, par exemple à l'intérieur d'un adaptateur (43), tel qu'un adaptateur Luer, et que la rondelle d'étanchéité (47) est formée dans le creux élargi.

6. Dispositif selon la revendication 5, caractérisé en ce que la rondelle d'étanchéité (47) remplit entièrement son creux de logement du côté de l'aiguille creuse.

7. Dispositif selon une des revendications précédentes, caractérisé en ce que la rondelle d'étanchéité (47) est maintenue par une bague de maintien (49).

8. Dispositif selon une des revendications précédentes, caractérisé en ce que la rondelle d'étanchéité (47) est maintenue par une soudure par ultrason annulaire spéciale.

9. Dispositif selon une des revendications précédentes, caractérisé en ce que la rondelle d'étanchéité (47) est de caractère auto-obturateur après avoir été percée.

10. Procédé de prélèvement de liquides, tissus ou similaires de l'intérieur d'une matière morte, caractérisé en ce qu'est introduite dans le corps jusqu'à l'endroit de prélèvement, l'extrémité distale d'un dispositif aspirateur qui présente une aiguille creuse ayant un tranchant à son extrémité distale et un mandrin adapté essentiellement au creux de l'aiguille creuse et un joint d'étanchéité, sous forme d'une rondelle d'étanchéité entièrement fermée, qui n'est percée que par le mandrin et placée à l'endroit opposé à l'extrémité distale de l'aiguille creuse et qu'ensuite le mandrin est retiré dans le corps creux, du moins partiellement, et qu'alors des liquide de tissus ou des échantillons de tissus sont aspirés.

11. Procédé selon la revendication 10, caractérisé en ce que lorsqu'on retire le mandrin l'aiguille creuse est légèrement poussée en avant.

12. Procédé selon la revendication 10 ou 11, caractérisé en ce qu'après l'enlèvement de l'aiguille creuse du corps un échantillon diopsique prélevé peut être éjecté par l'enfoncement du mandrin, éventuellement après une réintroduction au travers du joint d'étanchéité de l'aiguille creuse.

13. L'application d'un dispositif, réalisé comme dispositif d'aspiration en ce que dans une aiguille creuse (4), munié d'un tranchant à son extrémité distale (6) un mandrin (12) oblong, adapté de façon essentielle au creux de l'aiguille est introduit, qu'un joint d'étanchéité sous forme d'une rondelle d'étanchéité (47) entièrement fermée placée à l'endroit opposé à l'extrémité distale (6) de l'aiguille creuse (4), qui n'est percée que par le mandrin (12) pour le prélèvement je liquides, de tissus ou similaires de l'intérieur d'une matière morte.

FIG.1

FIG.2

FIG.3

FIG.4